# EUROPEAN PATENT SPECIFICATION

(11) **EP 1 179 067 B1**
(45) Date of publication and mention of the grant of the patent: **13.12.2006**
(21) Application number: 00935043.0
(22) Date of filing: 16.05.2000
(51) Int. Cl.: C12N 15/19, C12N 5/10, C07K 14/495, C07K 16/24, A61K 48/00, A61K 39/395, A61K 38/00

(54) **NEUROPROTECTIVE PROPERTIES OF GDF-15, A MEMBER OF THE TGF-BETA SUPERFAMILY**
NEUROPROTEKTIVE EIGENSCHAFTEN VON GDF-15, EIN MITGLIED DER TGF-BETA SUPERFAMILIE
PROPRIETES NEUROPROTECTRICES DU GDF-15, UN MEMBRE DE LA SUPERFAMILLE DE TGF-BETA

(30) Priority: 17.05.1999 EP 99109714; 29.07.1999 EP 99114853
(43) Date of publication of application: 13.02.2002
(73) Proprietor: BIOPHARM GESELLSCHAFT ZUR BIOTECHNOLOGISCHEN ENTWICKLUNG UND ZUM VERTRIEB VON PHARMAKA mbh, 69115 Heidelberg (DE)
(72) Inventor: UNSICKER, Klaus, D-69118 Heidelberg (DE); KRIEGLSTEIN, Kerstin, D-69118 Heidelberg (DE)
(74) Representative: Müller-Boré & Partner Patentanwälte
(86) International application number: PCT/EP2000/004445
(87) International publication number: WO 2000/070051

(56) References cited:
- WO-A-97/00958
- WO-A-98/11224
- WO-A2-00/65028
- BOOTCOV MR. ET AL.: "MIC-1, a novel macrophage inhibitory cytokine, is a divergent member of e TGF-beta superfamily." PROC NATL ACAD SCI U S A 1997 OCT 14;94(21):11514-9, XP002146474 cited in the application
- BÖTTNER M. ET AL.: "Expression of a novel member of the TGF-beta superfamily, growth/differentiation factor-15/macrophage-inhibiting cytokine-1 DF-15/MIC-1) in adult rat tissues." CELL TISSUE RES 1999 JUL;297(1):103-10, XP000937690

### SEQUENCE LISTING

<110> Biopharm Gesellschaft zur biotechnologischen ...
<120> Neuroprotective properties of GDF-15, a novel member of the TGF-β superfamily
<130> B 1991
<140> PCT/EP00/04445
   <141> 2000-05-16
<150> EP 99 109 714.8
   <151> 1999-05-17
<150> EP 99 114 853.7
   <151> 1999-07-29
<160> 7
<170> PatentIn Ver. 2.1
<210> 1
   <211> 888
   <212> DNA
   <213> Homo sapiens
<400> 1
   atgctcctgg tgttgctggt gctctcgtgg ctgccgcatg ggggcgccct gtctctggcc 60
   gaggcgagcc gcgcaagttt cccgggaccc tcagagttgc actccgaaga ctccagattc 120
   cgagagttgc ggaaacgcta cgaggacctg ctaaccaggc tgcgggccaa ccagagctgg 180
   gaagattcga acaccgacct cgtcccggcc cctgcagtcc ggatactcac gccagaagtg 240
   cggctgggat ccggcggcca cctgcacctg cgtatctctc gggccgccct tcccgagggg 300
   ctccccgagg cctcccgcct tcaccgggct ctgttccggc tgtccccgac ggcgtcaagg 360
   tcgtgggacg tgacacgacc gctgcggcgt cagctcagcc ttgcaagacc ccaggcgccc 420
   gcgctgcacc tgcgactgtc gccgccgccg tcgcagtcgg accaactgct ggcagaatct 480
   tcgtccgcac ggccccagct ggagttgcac ttgcggccgc aagccgccag ggggcgccgc 540
   agagcgcgtg cgcgcaacgg ggacgactgt ccgctcgggc ccgggcgttg ctgccgtctg 600
   cacacggtcc gcgcgtcgct ggaagacctg ggctgggccg attgggtgct gtcgccacgg 660
   gaggtgcaag tgaccatgtg catcggcgcg tgcccgagcc agttccgggc ggcaaacatg 720
   cacgcgcaga tcaagacgag cctgcaccgc ctgaagcccg acacggtgcc agcgccctgc 780
   tgcgtgcccg ccagctacaa tcccatggtg ctcttacaaa agaccgacac cggggtgtcg 840
   ctccagacct atgatgactt gttagccaaa gactgccact gcatatga            888
<210> 2
   <211> 339
   <212> DNA
   <213> Homo sapiens
<400> 2
   gcgcgcaacg gggacgactg tccgctcggg cccgggcgtt gctgccgtct gcacacggtc 60
   cgcgcgtcgc tggaagacct gggctgggcc gattgggtgc tgtcgccacg ggaggtgcaa 120
   gtgaccatgt gcatcggcgc gtgcccgagc cagttccggg cggcaaacat gcacgcgcag 180
   atcaagacga gcctgcaccg cctgaagccc gacacggtgc cagcgccctg ctgcgtgccc 240
   gccagctaca atcccatggt gctcttacaa aagaccgaca ccggggtgtc gctccagacc 300
   tatgatgact tgttagccaa agactgccac tgcatatga                        339
<210> 3
   <211> 295
   <212> PRT
   <213> Homo sapiens
<400> 3
<210> 4
   <211> 112
   <212> PRT
   <213> Homo sapiens
<400> 4
   Ala Arg Asn Gly Asp His Cys Pro Leu Gly Pro Gly Arg Cys Cys Arg
   1 5 10 15
   Leu His Thr Val Arg Ala Ser Leu Glu Asp Leu Gly Trp Ala Asp Trp
   20 25 30
   Val Leu Ser Pro Arg Glu Val Gln Val Thr Met Cys Ile Gly Ala Cys
   35 40 45
   Pro Ser Gln Phe Arg Ala Ala Asn Met His Ala Gln Ile Lys Thr Ser
   50 55 60
   Leu His Arg Leu Lys Pro Asp Thr Val Pro Ala Pro Cys Cys Val Pro
   65 70 75 80
   Ala Ser Tyr Asn Pro Met Val Leu Ile Gln Lys Thr Asp Thr Gly Val
   85 90 95
   Ser Leu Gln Thr Tyr Asp Asp Leu Leu Ala Lys Asp Cys His Cys Ile
   100 105 110
<210> 5
   <211> 13
   <212> PRT
   <213> Homo sapiens
<400> 5
   Met Pro Gly Gln Glu Leu Arg Thr Leu Asn Gly Ser Gln
   1 5 10
<210> 6
   <211> 15
   <212> PRT
   <213> Artificial Sequence
<220>
   <223> Description of Artificial Sequence: Peptide derived from the murine and rat C-terminal sequence of GDF-15
<400> 6
   His Arg Thr Asp Ser Gly Val Ser Leu Gln Thr Tyr Asp Asp Leu
   1 5 10 15
<210> 7
   <211> 15
   <212> PRT
   <213> Homo sapiens
<220>
   <221> PEPTIDE
   <222> (1)..(15)
   <223> Peptide corresponds to amino acids 273 to 287 of human pre-pro-mature GDF-15
<400> 7
   Gln Lys Thr Asp Thr Gly Val Ser Leu Gln Thr Tyr Asp Asp Leu
   1 5 10 15

## Description

The present invention relates to the use of a nucleic acid containing a nucleotide sequence encoding the primary amino acid sequence of a protein, e.g. a TGF-β-like protein, which is e.g. derived from neurons and glial cells, or a functionally active fragment thereof having at least a neurotrophic effect on DAergic neurons or functionally active homolog thereof having conservative amino acid substitutions and having at least a neurotrophic effect on DAergic neurons, optionally in combination with a pharmaceutically acceptable carrier and/or diluent, in the manufacture of a pharmaceutical composition for the prevention and/or treatment of neurodegenerative disorders in mammals or of a diagnostic kit for the detection of said disorders.

Members of the TGF-β superfamily are known for their important multifunctional implications in development and maintenance, such as the organization of the body plan, regulation of cell proliferation, differentiation, and cell survival. The still expanding TGF-β superfamily includes the TGF-β isoforms 1 to 5, activins, inhibins, bone morphogenetic proteins (BMPs), growth/differentiation factors (GDFs), mullerian-inhibiting substance, *Drosophila* decapentaplegic gene complex, *Xenopus* Vg-1 gene, and a growing subfamily of glial cell line-derived growth factors (GDNFs) and related proteins. All members of the TGF-β superfamily share several homologous structures. They are synthesized as large precursor molecules containing a biologically inactive pro-domain which can be secreted as a complex with the mature carboxyterminal portion. Furthermore, the mature bioactive proteins are generated by proteolysis using a characteristic cleavage site. Most notably, the mature carboxy-terminal segments contain a highly conserved cystein knot.

Bootcov et al. (Bootcov MR. et al., MIC-1, a novel macrophage inhibitory cytokine, is a divergent member of the TGF-β superfamily, Proc., Natl., Acad. Sci. USA, Vol. 94, pp. 11514-11519, Oct. 1997) describe the identification and expression of a novel transforming growth factor β (TGF-β) superfamily cytokine designated macrophage inhibitory cytokine-1 (MIC-1). Further, WO 97/00958 relates to a novel TGF-β like cytokine and to isolated polynucleotide molecules encoding this protein. Moreover, WO 98/11224 relates to a recombinant protein comprising the amino acid sequence of mature GF-2H which selectively inhibits the proliferation of androgen-independent prostate cancer cells when contacted therewith.

As TGF-β-like proteins are also implicated in the regulation of neuronal stem cell proliferation and maintenance of neurons there is a great demand for novel members of this protein family.

Accordingly, the technical problem underlying the present invention is to provide novel compounds relating to TGF-β-like proteins having neurotrophic activities which are suitable for the treatment and diagnosis of neurodegenerative disorders.

The solution to the above technical problem is achieved by providing the embodiments as characterized in the claims.

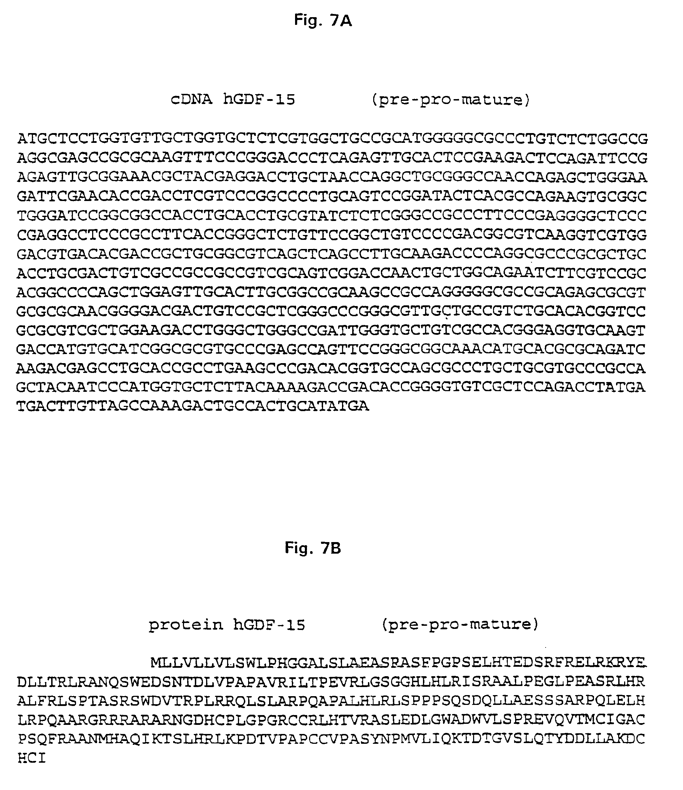

In particular, the present invention relates to the use of a nucleic acid containing a nucleotide sequence encoding the primary amino acid sequence of a protein comprising at least the primary amino acid sequence shown in Fig. 7B, the primary amino acid sequence shown in Fig. 8B, or amino acid residues 14 to 111 of the sequence shown in Fig. 8B, e.g. a TGF-β-like protein, which is e.g. derived from neurons and glial cells, or a functionally active fragment thereof having at least a neurotrophic effect on DAergic neurons or functionally active homolog thereof having conservative amino acid substitutions and having at least a neurotrophic effect on DAergic neurons or a vector containing at least the nucleic acid or a protein encoded by the nucleic acid, optionally in combination with a pharmaceutically acceptable carrier and/or diluent, in the manufacture of a pharmaceutical composition for the prevention and/or treatment of neurodegenerative disorders in mammals.

The terms "nucleic acid" and "nucleotide sequence" refer to endogenously expressed, semi-synthetic, synthetic or chemically modified nucleic acid molecules, preferably consisting substantially of deoxyribonucleotides and/or ribonucleotides and/or modified nucleotides. Further, the term "nucleotide sequence" may comprise exons, wherein the nucleotide sequence encodes the primary amino acid sequence and may be degenerated based on the genetic code. The term "primary amino acid sequence" refers to the sequence of amino acids irrespective of tertiary and quaternary protein structure.

The term "TGF-β-like protein" refers to proteins displaying the characteristics of the TGF-β superfamily, especially a conserved cystein rich motif, and comprises both the large precursor molecules containing a pro-domain as well as the mature bioactive proteins which are generated by proteolysis using a characteristic cleavage site.

The terms "functionally active derivative" and "functionally active part" refer to a proteinaceous compound exhibiting at least a neurotrophic effect on DAergic neurons. The functionally active form of the above-defined protein may be a monomeric, dimeric and/or oligomeric form, as well as a heterooligomeric form, e.g. a heterodimer, comprising at least two different monomers of TGF-β-like proteins having neurotrophic activity.

The expression "derived from neurons and glial cells" means that the gene coding for the protein is transcribed and/or translated in neurons and glial cells such as Purkinje cells and astrocytes such that the mRNA and/or the protein is detectable by methods known in the art such as *in situ* hybridization, RT-PCR, Northern or Western blotting.

The expression "neurotrophic effect on DAergic neurons" refers to a proteinaceous activity that may confer, by itself or in combination with other factors, survival and differentiation upon DAergic neurons within the nanomolar range or below.

In a preferred embodiment of the above defined use the neurons and glial cells are of mammalian origin, e.g. human, mouse or rat.

In a further preferred embodiment, the protein protects against neurodegenerative events. Such neurodegenerative events may be e.g. mediated by oxidative damage, free radicals, mediators or executors of neuronal death programs such as caspases, pro- and anti-apoptotic members of the bcl-2 family. A toxic radical damage may be mediated by iron, e.g. Fe-ions, NO and other radical donors. Therefore, the nucleic acid as defined above encodes a TGF-β-like protein which is able to protect DAergic neurons against intoxication by iron, which is suggested to cause Parkinson's disease (PD).

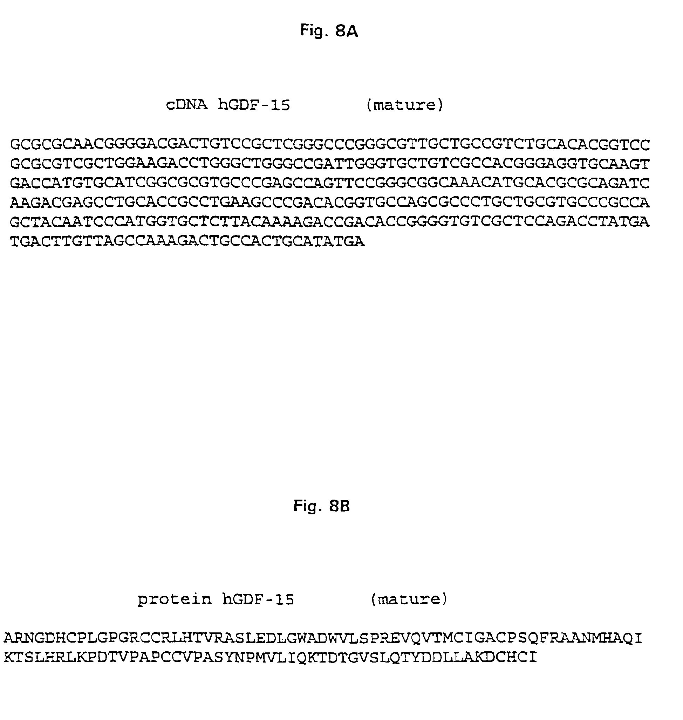

In a further preferred embodiment the nucleic acid used according to the present invention comprises at least the nucleotide sequence shown in Fig. 7A or the nucleotide sequence shown in Fig. 8A or nucleotides 40 to 333 of the nucleotide sequence shown in Fig. 8A or mutants of such nucleic acids leading to the expression of functionally active polypeptides. Examples of such mutations include deletions, insertions and substitutions of one or more nucleotides such as mutations which lead to conservative amino acid substitutions, e.g. such mutations in the range of nucleotides 40 to 333 of the nucleotide sequence shown in Fig. 8A, i.e. the region of the nucleotide sequence encoding the 7 Cysknot region which is highly conserved in TGF-β-like proteins.

A further subject of the present invention relates to a vector containing at least the nucleic acid as defined above. The term "vector" refers to a DNA and/or RNA replicon that can be used for the amplification and/or expression of the above defined nucleotide sequence. The vector may contain any useful control units such as promotors, enhancers, or other stretches of sequence within the 5' regions of the sequence serving for the control of its expression. The vector may additionally contain sequences within the 5' and/or 3' region of the nucleotide sequence, that encode amino acid sequences such as a His-tag which are useful for the detection and/or isolation of the protein encoded by the nucleotide sequence. Furthermore, the vector may contain sequence elements within the 5' and/or 3' region of the nucleotide sequence encoding amino acid sequences which serve for the targeting of the protein encoded by the nucleotide sequence to nerve tissues and/or for the penetration of the blood/brain barrier. Examples of suitable vectors are baculovirus vectors.

The nucleic acid or the vector used according to the present invention may be contained in a host organism. The term "host organism" comprises a virus, a bacterium such as *Escherichia coli,* a fungus, a plant, a non-human mammal or an insect or parts such as cells, e.g. Sf9 cells, thereof.

A modulation of the functional activity of the protein used according to the present invention may also be achieved by altering the expression of the nucleotide sequence of the above-defined nucleic acid as compared to the expression level in a normal cell. For example, an antisense nucleic acid masking the mRNA or a ribozyme cleaving the mRNA may be used to inhibit the expression. Alternatively, the efficiency of the promoter which regulates the expression of the nucleotide sequence of the above-defined nucleic acid may be influenced.

The nucleic acid, the vector, or the protein used as defined above may be produced e.g. by a method, comprising the steps of:
(a) cultivating a host organism in a suitable medium under suitable conditions; and
(b) isolating the desired product from the medium and/or the host organisms.

A preferred embodiment of the method for the production of the protein used according to the present invention uses bacteria such as *E. coli* as the host organsim. The expression of the protein may then lead to a functionally inactive form, e.g. of amorphous aggregates within the bacterium known in the art as "inclusion bodies". Therefore, the method may further comprise steps serving for the refolding and/or modification of the isolated protein into a functionally active form which may be a monomeric, dimeric or oligomeric form. In particular, this may be a method for the production of the biologically active dimeric form of the protein as defined above preferably GDF-15, from its denatured or otherwise non-native form. This result is achieved by the unexpected finding that considerable amounts of the desired dimeric products are obtained by subjecting the monomeric form of the protein used according to the present invention to refolding conditions. Thus, the present invention also relates to the use of dimeric biologically active GDF-15 which has been produced by the above-defined method.

A further embodiment of the present invention relates to the use of the nucleic acid or the vector or the protein or the antibody or the antagonist or the agonist as defined above, optionally in combination with a pharmaceutically acceptable carrier and/or diluent in the manufacture of a pharmaceutical composition. The pharmaceutical composition may be used for the prevention and/or treatment of neurodegenerative disorders in mammals, preferably in humans. Furthermore, therapeutic techniques for the treatment of disorders which are associated with the expression of the nucleotide sequence of the nucleic acid according to the present invention may be designed using the above-mentioned agents which are capable of regulating the expression of the nucleotide sequence of the above-defined nucleic acid, e.g. antisense nucleic acids, ribozymes and/or agents for influencing promoter activity. The neurodegenerative disorders are preferably acute and/or chronic neurological and psychological disorders, and may be caused by stroke, parkinson's disease, Alzheimer's disease or other dementias, infections of the CNS and psychiatric disorders associated with disturbances in CNS transmitter systems such as depression and schizophrenia.

In a further preferred embodiment, the pharmaceutical composition acccording to the present invention further comprises, in addition to the nucleic acid or the vector or the protein or the antibody or the antagonist or the agonist as defined above, one or more other agents having neurotrophic activity. Preferred agents are, e.g., cytokines or functionally active derivatives or parts thereof. Preferred cytokines used in the pharmaceutical composition according to the present invention may be selected from the group consisting of GDF such as GDF-5, GDF-6, GDF-7, GDF-8 and GDF-9, GDNF, TGF such as TGF-α or TGF-β, e.g. TGF-β1, TGF-β2 or TGF-β3, activin A, BMP such as BMP-2, BMP-4, BMP-6, or BMP-7, BMP-11, BMP-12, BDNF, NGF, neurotrophines such as NT-3 or NT-4, EGF, CNTF and FGF such as FGF-2. The term "GDNF" includes GDNF, neurturin and persephin.

A further subject of the present invention relates to the use of the nucleic acid, the vector, the protein and/or the antibody as defined above, in the manufacture of a diagnostic kit for the detection of neurodegenerative disorders and/or infections of the CNS such as meningitis, e.g. a bacterial meningitis, in mammals, preferably humans. Examples of other neurodegenerative disorders are as defined above.

The figures show:

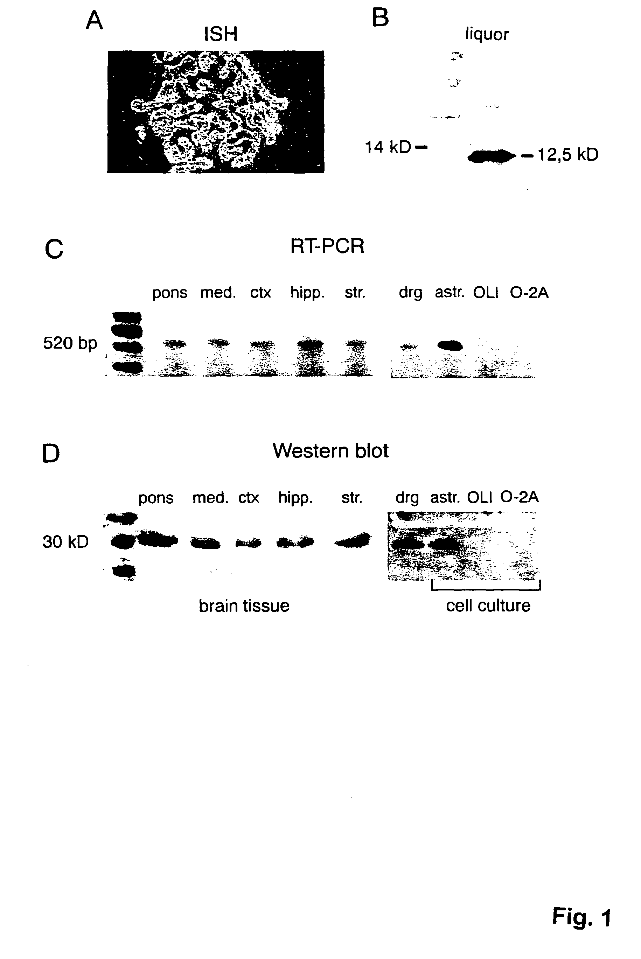

- Fig. 1: Localization of GDF-15 in the CNS. (A) Photographic image of an *in situ* hybridization of an adult rat choroid plexus performed with rat specific GDF-1 5 antisense-RNA probes. (B) Photographic image of an immunoblot analysis of human cerebrospinal fluid (CSF) under reducing conditions with purified GDF-15 antiserum. (C) RT-PCR of different PO rat brain regions (pons, medulla oblongata, cortex, hippocampus, striatum), dorsal root ganglia (DRG), cultured primary astrocytes (astr.), oligodendroglial cell line OLI-neu (OLI), and cultured oligodendroglial progenitors (O-2A). (D) Immunoblot analyis under native conditions of the corresponding brain areas and cells of (c) with purified GDF-15 antiserum.

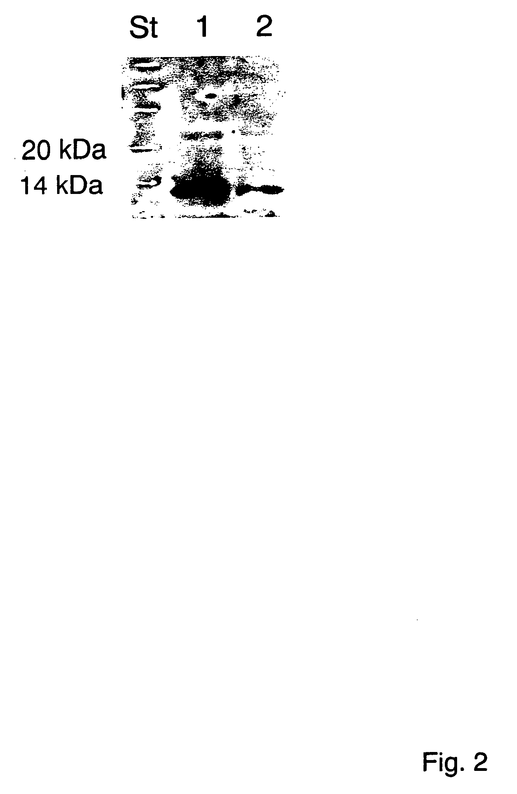

- Fig. 2: Image of Western blot analysis of GDF-15 in human CSF under reducing conditions. Molecular weight marker (St.). CSF sample of a patient with bacterial meningitis (lane 1). CSF sample of a control patient (lane 2).

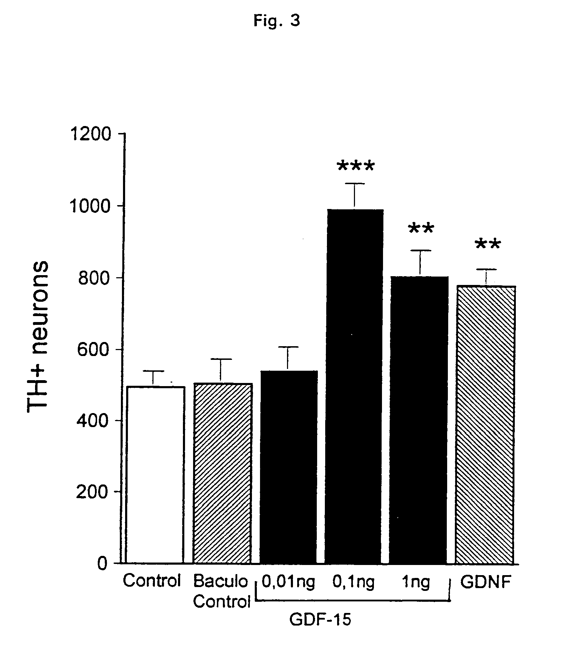

- Fig. 3: Graphic representation of experiments showing the survival effect of GDF-15 in mesencephalic neuron cultures. Numbers of surviving tyrosine hydroxylase (TH)-immunoreactive neurons of mesencephalic cultures (E15/DIV7) treated with medium only (control), purified lysate from uninfected Sf9 cells (baculo control), GDF-15 (0.01 to 1 ng/ml) purified from infected Sf9 cell lysate, and GDNF (10 ng/ml). Data are given as mean ± SEM (n=3), P-values derived from Student's t-test are ***P<0.001, **P<0.01 for increased survival as compared with control cultures.

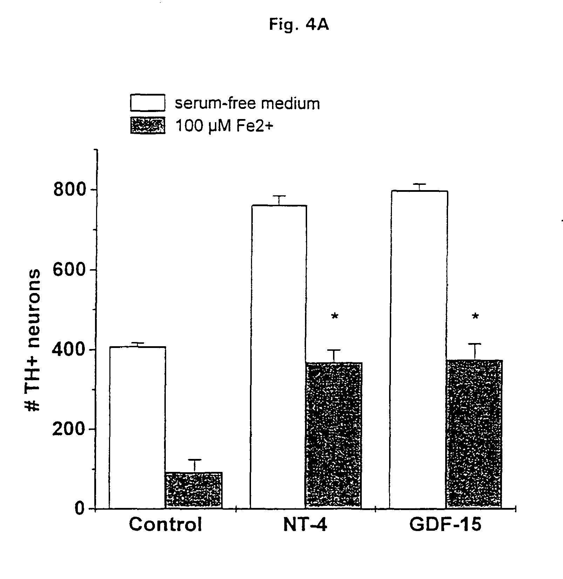

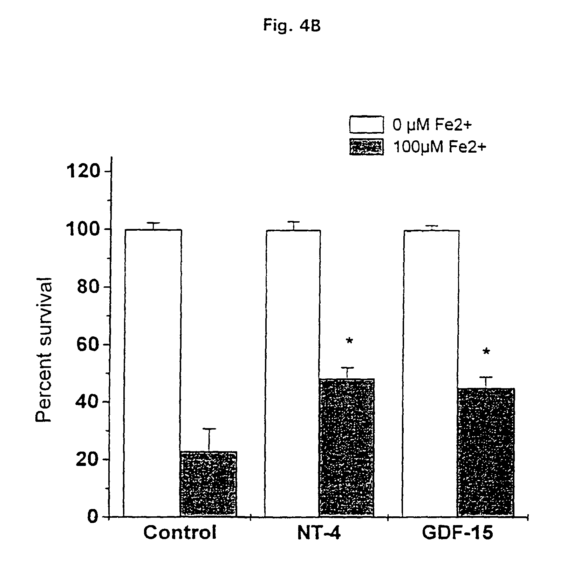

- Fig. 4: Protective effect of GDF-15 in Fe²⁺ (100 µM) treated cultures. (A) Graphic representation of numbers of surviving TH-immunoreactive neurons of mesencephalic cultures (E15/DIV7) treated with or without Fe²⁺ in medium only (control), in presence of NT-4 (10 ng/ml), and in presence of GDF-15 (10 ng/ml). (B) Graphic representation of the percentage of surviving TH-immunoreactive neurons of mesencephalic cultures (E15/DIV7) treated with Fe²⁺ in medium only (control), in presence of NT-4 (10 ng/ml), and in presence of GDF-15 (10 ng/ml). Values of cultures without addition of iron are set to 100%. Data are given as mean ± SEM (n=3), P-values derived from Student's t-test are * P < 0.05 for increased survival as compared with control cultures.

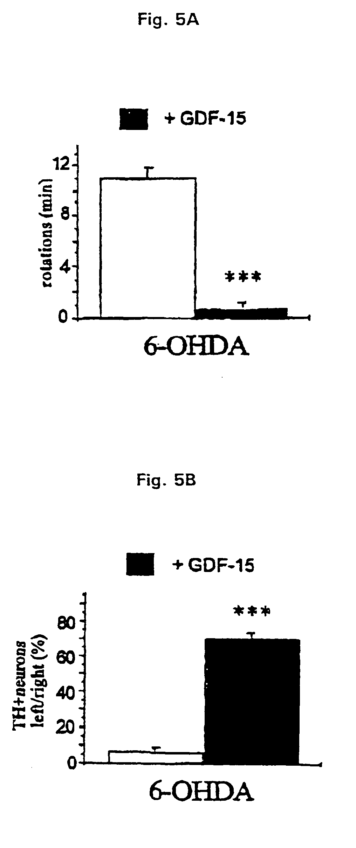

- Fig. 5: *In vivo* neurotrophic effects of GDF-15. (A) Graphic representation of amphetamine rotation data of rats with unilateral 6-OHDA (6-hydroxydopamine) lesions. Rotations per minute were monitored for 60 min beginning 5 min after amphetamine (5mg/kg i.p.) administration. (B) Graphic representation of counts of TH-positive neurons in SNpc. Values are given as percentage of TH-positive neurons of the lesioned as compared to the unlesioned side. Data are given as mean +/- SEM (n=4). P-values derived from Student's t-test are *P<0.05, **P<0.01, ***P<0.001.

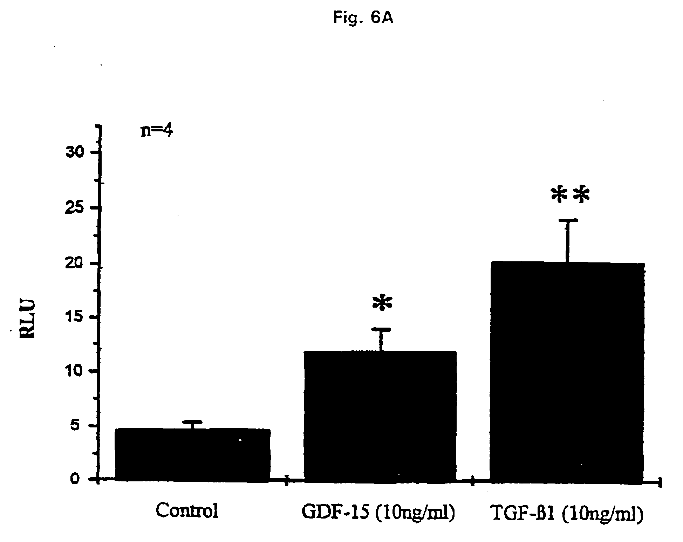

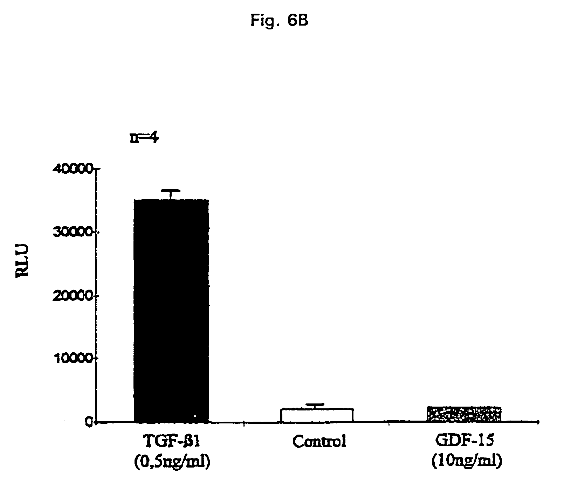

- Fig. 6: Signalling of GDF-1 5 through Smad proteins. (A) Graphic representation of experiments demonstrating the activation of the Smad Binding Element (SBE) by TGF-β1 and GDF-15 in transient transfected hFob cells. (B) Graphic representation of experiments showing that the PAI-1 promoter which is exclusively activated by TGF-β1 to -β3 in stable transfected MLEC cells does not respond to GDF-15.
- Fig. 7: (A) cDNA and (B) corresponding amino acid sequence of human pre-pro-mature GDF-15. Nucleotides and amino acids are abbreviated according to the international one letter codes.
- Fig. 8: (A) cDNA and (B) corresponding amino acid sequence of human mature GDF-15.

The following non-limiting example illustrates the invention:

### EXAMPLE

### Identification of GDF- 15

Using the conserved cystein knot motif of TGF-β-like proteins, a combined approach employing RT-PCR and library screening revealed the full-length cDNA sequence of a novel member of the TGF-β superfamily derived from neurons. The cDNA has the sequence shown in Fig. 7A corresponding to the amino acid sequence shown in Fig. 7B.

According to a possible alternative translation start codon which is located 39 nucleotides upstream from the first nucleotide of the sequence shown in Fig. 7A, the corresponding protein may also comprise 13 additional amino acids (MPGQELRTLNGSQ) N-terminal to the sequence shown in Fig. 7B.

The protein, which is named GDF-15, was recombinantly expressed using the baculovirus system. Furthermore, an antibody against a specific peptide derived from the murine and rat C-terminal sequence (HRTDSGVSLQTYDDL) has been developed. Due to the high homology of the corresponding region of the human sequence (QKTDTGVSLQTYDDL), this antibody recognizes also human GDF-15.

### Localization of GDF- 15 in the CNS

*In situ* hybridization with GDF-15 antisense RNA probes, RT-PCR as well as Western blot analyses were performed to study the distribution of GDF-15 in the CNS (Fig. 1). *In situ* hybridization revealed signals in neurons, especially Purkinje cells, in the cerebellum, and strong expression in the choroid plexus (Fig. 1 A) of newborn and adult rats. RT-PCR and Western blotting of samples taken from different regions of newborn and adult rat brains and peripheral nervous system extended these results by detecting mRNA and protein in pons, medulla oblongata, midbrain, striatum, hippocampus, cortex, and dorsal root ganglia (Fig. 1C, D). Highest levels of mRNA expression were found in the choroid plexus (Fig. 1 A). Antibodies raised against the above C-terminal peptide were used for Western blots. Analysis of samples of different brain areas of newborn rats revealed one distinct band at 31 kDa (Fig. 1D). The relative mass of cellular GDF-15 is in good agreement with the theoretical molecular weight of 31 kDa of the pro-protein.

As GDF-15 is abundant in the choroid plexus, the presence of the protein in CSF of healthy human subjects as well as patients with different neurological disorders was also tested. In contrast to the intracellular protein detected in brain samples, CSF samples revealed a single band at about 12 kDa under reducing conditions representing the secreted mature portion of GDF-15. Highest amounts of protein in CSF were seen in patients with bacterial meningitis (Fig. 2). Taken together these data provide evidence that GDF-15, a novel member of the TGF-β superfamily, is widely expressed in various regions of the CNS including CSF and peripheral nervous system. Furthermore, GDF-1 5 is significantly increased in the CSF of patients with inflammatory neurological disease providing the opportunity to employ antibodies to GDF-1 5 as diagnostic tools in neurological disease.

### Production of dimeric, biologically active GDF- 15

2 µg of monomeric GDF-15 protein (e.g. produced in bacteria such as *E. coli*) is dissolved in 2917.7 µl solubilisation buffer (1 M NaCl, 50 mM Tris-HCl, 50 mM EDTA, pH 9.5). To the thus dissolved protein, the following is added (resulting in a total volume of 3580 µl):
35.8 µl 100 mM oxidized Glutathion (GSSG)
35.8 µl 200 mM reduced Glutathion (GSH)
590.7 µl CHAPS (3-[(Cholamidopropyl)-dimethylamino]-1-propane sulfonate)

After incubation at 20 to 22°C for 48 h, more than 80%, typically 90%, of the monomeric protein is refolded into the desired dimeric product. The separation of the dimer is performed by standard chromatographic methods such as reverse phase HPLC.

### Functional studies using recombinant human GDF 15

Using the baculovirus system, the mature part of the human recombinant GDF-15 protein was expressed in Sf9 cells. However, the same results in all functional studies are obtained when using recombinant human GDF-15 expressed in bacteria which has been renatured by the above-described refolding method. Western blot showed the monomeric or dimeric form of the recombinant protein under reducing and non-reducing conditions, respectively. Following purification, the protein was tested for its survival effects on rat embryonic midbrain DAergic neurons. Addition of recombinant GDF-15 to cultures of E14 midbrain cells augmented numbers of surviving tyrosine hydroxylase (TH)-positive neurons after 7 days *in vitro* compared to control cultures (Fig. 3). The dopaminotrophic effect of GDF-15 is comparable to the documented survival promoting activity of other members of the TGF-β superfamily and the neutrophin family (e.g. TGF-β, GDNF-subfamily members, or BDNF). Analysis of midbrain cultures using immunocytochemistry and antibodies to the astrocyte-specific intermediate filament protein GFAP and assays for cell proliferation provided evidence that GDF-1 5 application did not exert its survival promoting effect through numerically increasing cells and promoting maturation of astrocytes, a well-established source of neurotrophic factors. This provides evidence that GDF-15 affects dopaminergic neurons directly rather than indirectly, as shown for FGF-2 or BMPs.

In order to investigate whether GDF-15 is also able to protect DAergic neurons against a likely cause of PD, i.e. iron intoxication, its effects on iron-intoxicated mesencephalic neurons was examined (Fig. 4A, B). Exposure of cultures to iron (Fe²⁺) caused a 80% reduction in neuronal survival compared to untreated control cultures. Cell losses were reduced to 50% when cultures were co-treated with Fe²⁺ and GDF-15. These data strongly suggest that GDF-15 protects DAergic neurons against iron-mediated (oxidative) damage. The data also support the use of GDF-1 5 as an agent to prevent or slow down neurodegenerative events mediated by free radicals, oxidative stress, mediators and executors of neuronal death programs.

Furthermore, it was established that GDF-15 also protects lesioned DAergic midbrain neurons *in vivo.* The nigrostriatal system of adult rats was lesioned by an unilateral injection of 6-hydroxydopamine (6-OHDA) just above the left substantia nigra (SN). The results of these experiments are shown in Tables 1A and B, respectively. The data shown in Table 1B are also represented graphically in Fig. 5A.

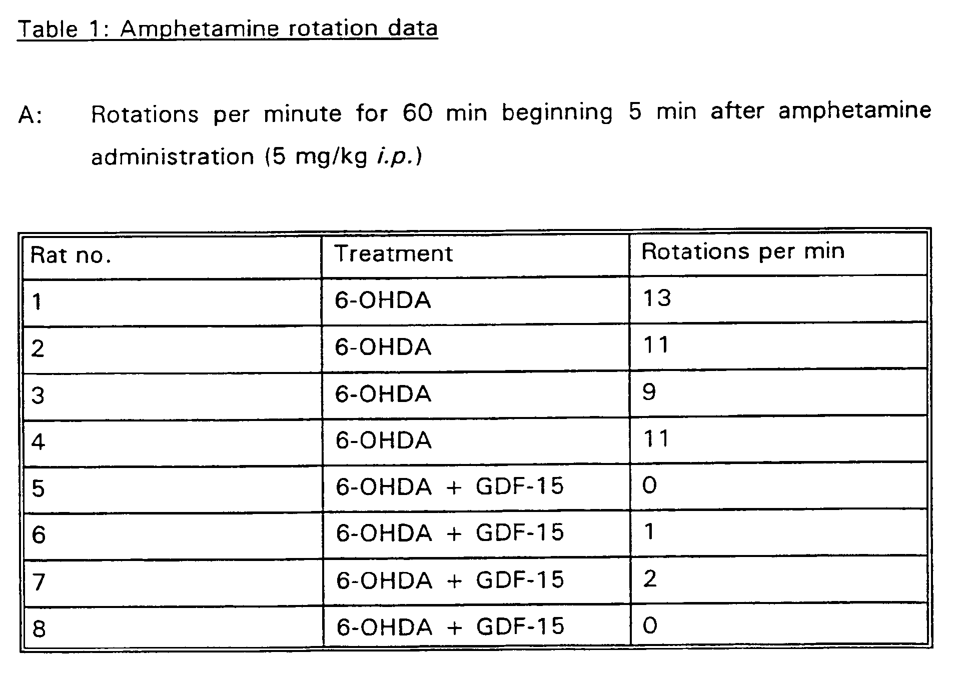

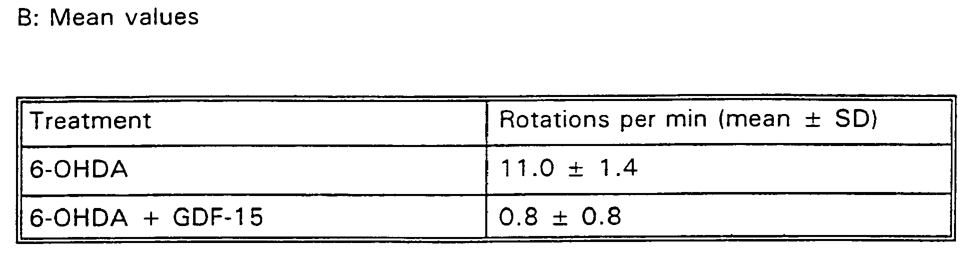

All rats displayed the typical features of amphetamine challenge, such as stereotypy and piloerection. Rats which had been treated with 6-OHDA only showed ratation rates of 11.0 ± 1.41 (mean ± SD), indicating at least 95% depletion of the nigrostriatal pathway (Ungerstedt at al. (1970), Brain. Res., 24, 485-493). In contrast, rats which were also treated with GDF-15 rotated at very low rates (0.75 ± 0.83), showing that this protein effectively prevented 6-OHDA-induced depletion of dopamine in the left striatum; cf. also Fig. 5A.

Furthermore, in order to confirm that the above prevention of 6-OHDA-induced dopamine depletion in the left striatum is due to a neuroprotective effect of GDF-15 on neurons in the SN, the SN pars compacta (SNpc) was analysed immunocytochemically. Counts of TH-positive neurons in the SNpc measured at three individual levels are shown in Table 2A and the mean values for each rat are given in Table 2B, respectively. The overall mean values for the 6-OHDA-treated (n = 4) and for the rats which were co-treated with 6-OHDA and GDF-1 5 (n = 4) are shown in Table 2C. The data shown in Table 2C are also represented graphically in Fig. 5B. The results show that the co-treatment of the rats with 6-OHDA and GDF-15 led to a 10-fold increase in the count of TH-positive neurons in the left striatum compared to the treatment with 6-OHDA alone. Therefore, GDF-15 prevents 6-OHDA-induced depletion of dopamine in the left striatum due to its strong neuroprotective effect on TH-immunoreactive neurons.

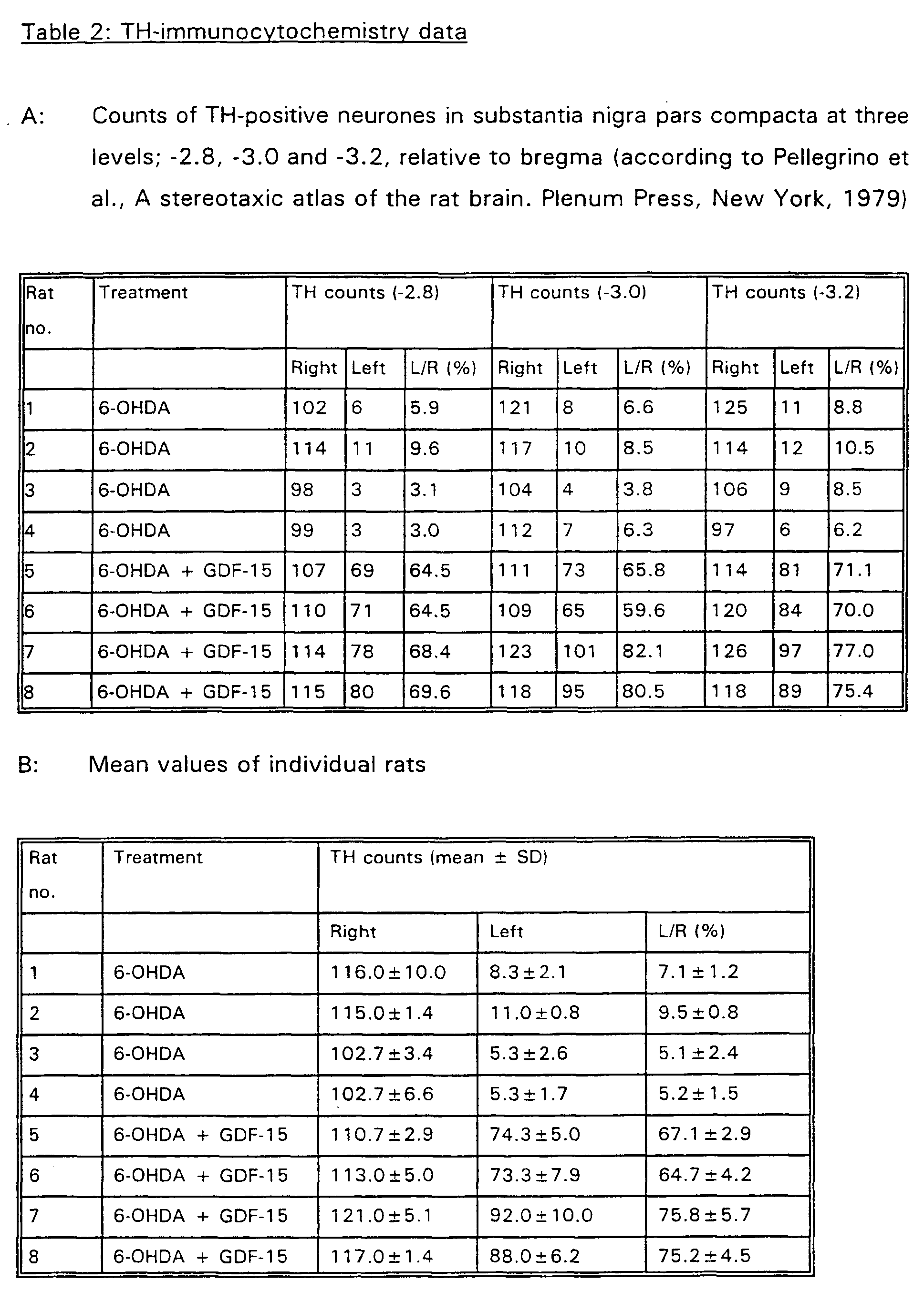

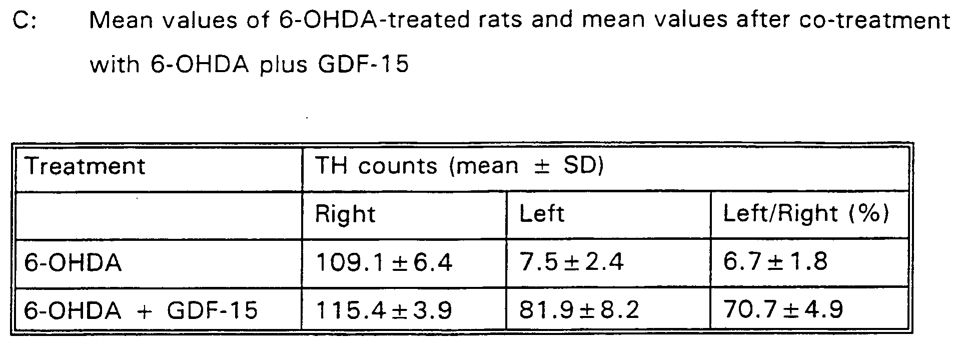

In summary, the above *in vivo* studies demonstrate that injections of GDF-15 immediately prior to 6-OHDA above the left SN and into the left lateral ventricle prevented 6-OHDA-induced pathological rotation behavior (Fig. 5A) and significantly reduced losses of DAergic SN neurons (Fig. 5B). Together, these data show that GDF-15 can be profitably employed to ameliorate consequences of nigrostriatal degeneration in Parkinson's disease.

Using the plasmid Smad Binding Element (pSBE) which is activated by TGF-β1, OP-1 (also referred to as BMP-7), activin, BMP-2 and GDF-5, the further question was addressed as to whether GDF-15 is able to induce intracellular signal transduction through Smad proteins. Transient transfection of the human osteoblast cell line (hFob) with SBE showed that GDF-15 administration increased the luciferase signal (Fig. 6A). These results demonstrate that GDF-15 activates the Smad responsive promoter element of the reporter gene construct. In a further experiment the inducibility of the Plasminogene Activator Inhibitor promoter (PAI) in stable transfected Mink Lung Epithelial Cells (MLEC) by GDF-15 was tested. The MLEC assay, which is exclusively sensitive for TGF-β1, -β2, and -β3, revealed no effect of GDF-1 5 (Fig. 6B). Since Smad2 and Smad3 phosphorylation is specifically associated with the TGF-β-mediated activation of TGF-β receptors, it is concluded that GDF-15 seems not to signal through the Smad2/3 pathway. With regard to the GDF-15-dependent activation of SBE, which is a response element for both, the Smad2/3, and the BMP-mediated Smad1/5 pathway, it appears that GDF-1 5 exerts its cellular effects by binding to BMP-like receptors.

### Summary

In conclusion, a novel neurotrophic molecule derived from neuron cells belonging to the TGF-β superfamily, GDF-15, was discovered, cloned, expressed and functionally characterized.

In the nervous system, GDF-15 mRNA and protein can be detected, e.g. in midbrain, striatum and in cortex, but highest levels of the mRNA and the protein are found in the choroid plexus and spinal fluid (CSF), respectively. Interestingly, levels of protein in CSF are increased in certain neurological disorders, e.g. in patients with bacterial meningitis. In order to elucidate its functions, the mature form of human GDF-15 was recombinantly expressed using a baculovirus expression system. Expression resulted in the synthesis of the biologically active dimeric form of the protein. *In vitro* experiments using dissociated cell cultures of embryonic rat midbrain neurons revealed that GDF-15 can act as a neurotrophic factor for DAergic midbrain neurons which degenerate in Parkinson's disease (PD). GDF-15 is also able to protect these neurons against intoxication by iron, which may be causal to PD. Furthermore, it could be demonstrated that GDf-15 also exhibits its neuroprotective effect *in vivo.* Concerning the signalling pathway GDF-15 acts upon, it was established that GDF-15 is able to induce intracellular signal transduction through Smad proteins.

Therefore, it can be concluded that GDF-15 has important functions in the developing, mature, and lesioned brain involving options to use GDF-15 for the treatment and diagnosis of acute and chronic neurological and psychological disorders, such as stroke, Alzheimer's disease and other demetias, and psychiatric disorders associated with disturbances in CNS transmitter systems.

### Methods for in vivo studies demonstrating the protective effect of GDF- 15 on 6-OHDA-lesioned nigrostriatal neurons

Adult female Wistar rats were anaesthetised using ketamine (75 mg/kg i.p.) and xylazinum (15 mg/kg i.p.) and placed in a Kopf stereotaxic frame. GDF-15 was used at a final concentration of 2 *µ*g/*µ*l in 10 mM phosphate-buffered saline (PBS), pH 7,4. Four rats received injections of 20 *µ*g GDF-1 5 just above the left substantia nigra (SN) and 20 *µ*g GDF-1 5 into the left lateral ventricle (LV). This was followed immediately by an injection of 6-hydroxydopamine hydrobromide (8 *µ*g as the free base in 4 *µ*l 0.9% saline with 0.1 % ascorbic acid) into the left medial forebrain bundle (MFB). Four additional rats received 6-OHDA only. Stereotaxic co-ordinates (Pellegrino et al. A stereotaxic atlas of the rat brain. Plenum Press, New York, 1979) were as follows: AP -3.0, LV + 2.5, DV -8.5 for the SN; AP + 1.0, LV + 1.2, DV -3.5 for the LV; AP -2.2, LV + 1.5, DV -7.9 for the MFB. All rats were tested behaviourally at seven days after surgery. Ipsilateral rotations were counted over a 60 min period beginning 5 min after (+)-ampthetamine sulphate administration (5 mg/kg, i.p.). At ten days after surgery, all rats were terminally anaesthetised with chloroform/ether and perfused intracardially with 200 ml of cold 0.1 M phosphate-buffered saline (PBS), pH 7.4, containing 500 Units heparin, followed by 300 ml freshly prepared 4% paraformaldhyde in PBS. Brains were removed and placed in 4% paraformaldehyde in PBS overnight, cryoprotected in 30% sucrose in PBS and then frozen. Serial 30 *µ*m coronal cryosections through the SN pars compacta (SNpc) were cut and stained immunocytochemically for tyrosine hydroxylase (TH). Sections were incubated in blocking solution (3% normal goat serum, 0.2% Triton X-100 in PBS) overnight at 4°C, then in a 1:2000 solution of rabbit antiserum to TH (Affiniti Labs, U.K.) in blocking solution overnight at 4°C. Sections were washed five times in PBS containing 0.02% Triton X-100, then incubated in a solution of 1:1000 horse radish peroxidase-linked anti-rabbit IgG (Vector Labs) overnight at 4°C. After washing as before, TH immunostaining was visualised using 3,3'-diaminobenzidine as the chromogen. Sections were mounted onto gelatinised slides, dehydrated in alcohol, cleared in xylene and mounted in DePeX^{®} (Bioproducts, Heidelberg, Germany). TH-immunoreactive neurons were counted in the SNpc on both sides of the brain at each of three levels; -2.8, -3.0, -3.2, with respect to bregma (Pellegrino et al., 1979).

## Claims

1. Use of a nucleic acid containing a nucleotide sequence encoding the primary amino acid sequence of a protein comprising at least the primary amino acid sequence shown in Fig. 7B, the primary amino acid sequence shown in Fig. 8B, or amino acid residues 14 to 111 of the sequence shown in Fig. 8B or a functionally active fragment thereof having at least a neurotrophic effect on DAergic neurons or functionally active homolog thereof having conservative amino acid substitutions and having at least a neurotrophic effect on DAergic neurons or a vector containing at least the nucleic acid or a protein encoded by the nucleic acid, optionally in combination with a pharmaceutically acceptable carrier and/or diluent, in the manufacture of a pharmaceutical composition for the prevention and/or treatment of neurodegenerative disorders in mammals.

2. The use according to claim 1, wherein the protein or functionally active derivative or part thereof as defined in claim 1 is a monomer, dimer, oligomer, or heterooligomer.

3. The use according to claim 1 or 2, wherein the protein as defined in claim 1 protects against neurodegenerative events.

4. The use according to claim 3, wherein the neurodegenerative event is mediated by oxidative damage and/or free radical damage and/or mediators and/or executors of neuronal death programs.

5. The use according to claim 4, wherein the mediators of the free radical damage are selected from the group consisting of iron, NO donors, and other free radical donors, and the mediators and executors of neuronal death programs are selected from the group consisting of caspases and pro- and anti-apoptotic members of the bcl-2 family.

6. The use according to any one of claims 1 to 5, wherein the nucleic acid comprises at least the nucleotide sequence shown in Fig. 7A or the nucleotide sequence shown in Fig. 8A or nucleotides 40 to 333 of the nucleotide sequence shown in Fig. 8A or mutants thereof having a deletion, insertion and/or substitution of one nucleotide, said mutants leading to the expression of a functional polypeptide which has at least a neurotrophic effect on DAergic neurons.

7. The use according to any one of claims 1 to 5, wherein the protein encoded by the nucleic acid comprises at least the primary amino acid sequence shown in Fig. 7B or the primary amino acid sequence shown in Fig. 8B or amino acid residues 14 to 111 of the sequence shown in Fig. 8B as well as homologs thereof having conservative amino acid substitutions.

8. The use according to any one of claims 1 to 7, wherein the mammal is a human.

9. The use according to any one of claims 1 to 8, wherein the neurodegenerative disorders are selected from the group of acute and/or chronic neurological and psychological disorders.

10. The use of claim 9, wherein the neurological and psychological disorders are caused by stroke, Parkinson's disease, Alzheimer's disease or other dementias, infections of the CNS and psychiatric disorders associated with disturbances in CNS transmitter systems.

11. The use according to claim 10, wherein the psychiatric disorders are selected from the group consisting of depression and schizophrenia.

12. The use according to any one of claims 1 to 11 further comprising one or more agents having neurotrophic activity or functionally active derivatives or parts thereof.

13. The use according to claim 12, wherein the agent is a cytokine.

14. The use according to claim 13, wherein the cytokine is selected from the group consisting of GDF, GDNF, TGF, activins, BMP, BDNF, NGF, EGF, CNTF and FGF.

15. Use of a nucleic acid containing a nucleotide sequence encoding the primary amino acid sequence of a protein comprising at least the primary amino acid sequence shown in Fig. 7B, the primary amino acid sequence shown in Fig. 8B, or amino acid residues 14 to 111 of the sequence shown in Fig. 8B or a functionally active fragment thereof having at least a neurtrophic effect on DAergic neurons or functionally active homolog thereof having conservative amino acid substitutions and having at least a neurotrophic effect on DAergic neurons and/or a vector containing at least the nucleic acid and/or a protein encoded by the nucleic acid and/or an antibody or a functional fragment thereof directed against the protein, for the manufacture of a diagnostic kit for the detection of neurodegenerative disorders in mammals.

16. The use according to claim 15, wherein the protein or functionally active derivative or part thereof as defined in claim 15 is a monomer, dimer, oligomer, or heterooligomer.

17. The use according to claim 15 or 16, wherein the protein as defined in claim 15 protects against neurodegenerative events.

18. The use according to claim 17, wherein the neurodegenerative event is mediated by oxidative damage and/or free radical damage and/or mediators and/or executors of neuronal death programs.

19. The use according to claim 18, wherein the mediators of the free radical damage are selected from the group consisting of iron, NO donors, and other free radical donors, and the mediators and executors of neuronal death programs are selected from the group consisting of caspases and pro- and anti-apoptotic members of the bcl-2 family.

20. The use according to any one of claims 15 to 19, wherein the nucleic acid comprises at least the nucleotide sequence shown in Fig. 7A or the nucleotide sequence shown in Fig. 8A or nucleotides 40 to 333 of the nucleotide sequence shown in Fig. 8A or mutants thereof having a deletion, insertion and/or substitution of one nucleotide, said mutants leading to the expression of a functional polypeptide which has at least a neurotrophic effect on DAergic neurons.

21. The use according to any one of claims 15 to 20, wherein the protein encoded by the nucleic acid comprises at least the primary amino acid sequence shown in Fig. 7B or the primary amino acid sequence shown in Fig. 8B or amino acid residues 14 to 111 of the sequence shown in Fig. 8B as well as homologs thereof having conservative amino acid substitutions.

22. The use according to any one of claims 19 to 21, wherein the mammal is a human.

## Patentansprüche

1. Verwendung einer Nukleinsäure, enthaltend eine Nukleotidsequenz, kodierend die primäre Aminosäuresequenz eines Proteins, umfassend mindestens die in Fig. 7B gezeigte primäre Aminosäuresequenz, die in Fig. 8B gezeigte primäre Aminosäuresequenz oder die Aminosäurereste 14 bis 111 der in Fig. 8B gezeigten Sequenz oder ein funktionell aktives Fragment davon mit mindestens einem neurotrophen Effekt auf dopaminerge (DA-erge) Neuronen oder ein funktionell aktives Homolog davon mit konservativen Aminosäuresubstitutionen und mit mindestens einem neurotrophen Effekt auf DA-erge Neuronen oder einen Vektor, enthaltend mindestens die Nukleinsäure oder ein Protein, kodiert durch die Nukleinsäure, gegebenenfalls in Verbindung mit einem pharmazeutisch verträglichen Träger und/oder Verdünnungsmittel, in der Herstellung einer pharmazeutischen Zusammensetzung für die Prävention und/oder Behandlung von neurodegenerativen Störungen in Säugern.

2. Verwendung nach Anspruch 1, wobei das Protein oder das funktionell aktive Derivat oder ein Teil davon, wie in Anspruch 1 definiert, ein Monomer, Dimer, Oligomer oder Heterooligomer ist.

3. Verwendung nach Anspruch 1 oder 2, wobei das in Anspruch 1 definierte Protein gegen neurodegenerative Ereignisse schützt.

4. Verwendung nach Anspruch 3, wobei das neurodegenerative Ereignis durch oxidative Schädigung und/oder Schädigung durch freie Radikale und/oder Vermittler und/oder Träger von neuronalen Todesprogrammen vermittelt wird.

5. Verwendung nach Anspruch 4, wobei die Vermittler der Schädigung durch freie Radikale aus der Gruppe, bestehend aus Eisen, NO-Donatoren und anderen Donatoren für freie Radikale, ausgewählt sind, und die Vermittler und Träger der neuronalen Todesprogramme aus der Gruppe, bestehend aus Caspasen und pro- und anti-apoptotischen Vertretern der bcl-2-Familie, ausgewählt sind.

6. Verwendung nach einem der Ansprüche 1 bis 5, wobei die Nukleinsäure mindestens die in Fig. 7A gezeigte Nukleotidsequenz oder die in Fig. 8A gezeigte Nukleotidsequenz oder die Nukleotide 40 bis 333 der in Fig. 8A gezeigten Nukleotidsequenz oder Mutanten davon mit einer Deletion, Insertion und/oder Substitution eines Nukleotids umfaßt, wobei die Mutanten zu der Expression eines funktionellen Polypeptides führen, welches mindestens einen neurotrophen Effekt auf DA-erge Neuronen aufweist.

7. Verwendung nach einem der Ansprüche 1 bis 5, wobei das durch die Nukleinsäure kodierte Protein mindestens die in Fig. 7B gezeigte primäre Aminosäuresequenz oder die in Fig. 8B gezeigte primäre Aminosäuresequenz oder die Aminosäurereste 14 bis 111 der in Fig. 8B gezeigten Sequenz, sowie Homologe davon mit konservativen Aminosäuresubstitutionen, umfaßt.

8. Verwendung nach einem der Ansprüche 1 bis 7, wobei der Säuger ein Mensch ist.

9. Verwendung nach einem der Ansprüche 1 bis 8, wobei die neurodegenerativen Störungen aus der Gruppe von akuten und/oder chronischen neurologischen und psychologischen Störungen ausgewählt sind.

10. Verwendung nach Anspruch 9, wobei die neurologischen und psychologischen Störungen durch Schlaganfall, Parkinson-Krankheit, Alzheimer-Krankheit oder andere Demenzerkrankungen, Infektionen des ZNS und psychiatrische Störungen, die mit Störungen im ZNS-Transmittersystem verbunden sind, verursacht sind.

11. Verwendung nach Anspruch 10, wobei die psychiatrischen Störungen aus der Gruppe, bestehend aus Depression und Schizophrenie, ausgewählt sind.

12. Verwendung nach einem der Ansprüche 1 bis 11, weiter umfassend ein oder mehrere Mittel mit neurotropher Aktivität oder funktionell aktive Derivate oder Teile davon.

13. Verwendung nach Anspruch 12, wobei das Mittel ein Cytokin ist.

14. Verwendung nach Anspruch 13, wobei das Cytokin aus der Gruppe, bestehend aus GDF, GDNF, TGF, Aktivinen, BMP, BDNF, NGF, EGF, CNTF und FGF, ausgewählt ist.

15. Verwendung einer Nukleinsäure, enthaltend eine Nukleotidsequenz, kodierend die primäre Aminosäuresequenz eines Proteins, umfassend mindestens die in Fig. 7B gezeigte primäre Aminosäuresequenz, die in Fig. 8B gezeigte primäre Aminosäuresequenz oder die Aminosäurereste 14 bis 111 der in Fig. 8B gezeigten Sequenz oder ein funktionell aktives Fragment davon mit mindestens einem neurotrophen Effekt auf dopaminerge (DA-erge) Neuronen oder ein funktionell aktives Homolog davon mit konservativen Aminosäuresubstitutionen und mit mindestens einem neurotrophen Effekt auf DA-erge Neuronen und/oder einen Vektor, enthaltend mindestens die Nukleinsäure und/oder ein Protein, kodiert durch die Nukleinsäure, und/oder einen Antikörper oder ein funktionelles Fragment davon, welches gegen das Protein gerichtet ist, für die Herstellung eines diagnostischen Kits für die Detektion von neurodegenerativen Störungen in Säugern.

16. Verwendung nach Anspruch 15, wobei das Protein oder das funktionell aktive Derivat oder ein Teil davon, wie in Anspruch 15 definiert, ein Monomer, Dimer, Oligomer oder Heterooligomer ist.

17. Verwendung nach Anspruch 15 oder 16, wobei das in Anspruch 15 definierte Protein gegen neurodegenerative Ereignisse schützt.

18. Verwendung nach Anspruch 17, wobei das neurodegenerative Ereignis durch oxidative Schädigung und/oder Schädigung durch freie Radikale und/oder Vermittler und/oder Träger von neuronalen Todesprogrammen vermittelt wird.

19. Verwendung nach Anspruch 18, wobei die Vermittler der Schädigung durch freie Radikale aus der Gruppe, bestehend aus Eisen, NO-Donatoren und anderen Donatoren für freie Radikale, ausgewählt sind, und die Vermittler und Träger der neuronalen Todesprogramme aus der Gruppe, bestehend aus Caspasen und pro- und anti-apoptotischen Vertretern der bcl-2-Familie, ausgewählt sind.

20. Verwendung nach einem der Ansprüche 15 bis 19, wobei die Nukleinsäure mindestens die in Fig. 7A gezeigte Nukleotidsequenz oder die in Fig. 8A gezeigte Nukleotidsequenz oder die Nukleotide 40 bis 333 der in Fig. 8A gezeigten Nukleotidsequenz oder Mutanten davon mit einer Deletion, Insertion und/oder Substitution eines Nukleotids umfaßt, wobei die Mutanten zu der Expression eines funktionellen Polypeptids führen, welches mindestens einen neurotrophen Effekt auf DA-erge Neuronen aufweist.

21. Verwendung nach einem der Ansprüche 15 bis 20, wobei das durch die Nukleinsäure kodierte Protein mindestens die in Fig. 7B gezeigte primäre Aminosäuresequenz oder die in Fig. 8B gezeigte primäre Aminosäuresequenz oder die Aminosäurereste 14 bis 111 der in Fig. 8B gezeigten Sequenz, sowie Homologe davon mit konservativen Aminosäuresubstitutionen, umfaßt.

22. Verwendung nach einem der Ansprüche 19 bis 21, wobei der Säuger ein Mensch ist.

## Revendications

1. Utilisation d'un acide nucléique contenant une séquence nucléotidique codant pour la séquence primaire d'acides aminés d'une protéine, comprenant au moins la séquence primaire d'acides aminés présentée sur la Figure 7B, la séquence primaire d'acides aminés présentée sur la Figure 8B, ou les résidus d'acides aminés 14 à 111 de la séquence présentée sur la Figure 8B ou un fragment fonctionnellement actif de ces dernières, ayant au moins un effet neurotrophique sur les neurones DAergiques ou leurs homologues fonctionnellement actifs, ayant des substitutions d'acides aminés conservatives et ayant au moins un effet neurotrophique sur les neurones DAergiques, ou d'un vecteur contenant au moins l'acide nucléique ou une protéine codant par l'acide nucléique, en option en combinaison avec un excipient et/ou un diluant acceptable d'un point de vue pharmaceutique, pour préparer une composition pharmaceutique destinée à la prévention et/ou au traitement de troubles neurodégénératifs chez les mammifères.

2. Utilisation selon la revendication 1, pour laquelle la protéine ou son dérivé fonctionnellement actif ou la partie de cette dernière, telle que définie dans la revendication 1, est un monomère, un dimère, un oligomère ou un hétérooligomère.

3. Utilisation selon la revendication 1 ou 2, pour laquelle la protéine telle que définie dans la revendication 1 assure une protection contre les événements neurodégénératifs.

4. Utilisation selon la revendication 3, pour laquelle l'événement neurodégénératif est provoqué par un dommage oxydatif et/ou un dommage occasionné par les radicaux libres et/ou les médiateurs et/ou les exécuteurs des programmes de mort neuronale.

5. Utilisation selon la revendication 4, pour laquelle les médiateurs des dommages provoqués par des radicaux libres sont choisis dans l'ensemble consistant en le fer, les donneurs de NO et d'autres donneurs de radicaux libres, et les médiateurs et exécuteurs des programmes de mort neuronale sont choisis dans l'ensemble consistant en les caspases et les membres pro- et anti-apoptotiques de la famille bcl-2.

6. Utilisation selon l'une quelconque des revendications 1 à 5, pour laquelle l'acide nucléique comprend au moins la séquence nucléotidique présentée sur la Figure 7A ou la séquence nucléotidique présentée sur la Figure 8A ou les nucléotides 40 à 333 de la séquence nucléotidique présentée sur la Figure 8A ou leurs mutants ayant une délétion, une insertion et/ou une substitution non nucléotide, lesdits mutants conduisant à l'expression d'un polypeptide fonctionnel ayant au moins un effet neurotrophique sur les neurones DAergiques.

7. Utilisation selon l'une quelconque des revendications 1 à 5, pour laquelle la protéine codée par l'acide nucléique comprend au moins la séquence primaire d'acides aminés présentée sur la Figure 7B ou la séquence primaire d'acides aminés présentée sur la Figure 8B ou les résidus d'acides aminés 14 à 111 de la séquence présentée sur la Figure 8B, ainsi que leurs homologues ayant des substitutions conservatives d'acides aminés.

8. Utilisation selon l'une quelconque des revendications 1 à 7, pour laquelle le mammifère est un humain.

9. Utilisation selon l'une quelconque des revendications 1 à 8, pour laquelle les troubles neurodégénératifs sont choisis dans l'ensemble des troubles neurologiques et psychologiques aigus et/ou chroniques.

10. Utilisation selon la revendication 9, pour laquelle les troubles neurologiques et psychologiques sont provoqués par un accident vasculaire cérébral, une maladie de Parkinson, une maladie d'Alzheimer ou d'autres démences, des infections du SNC et des troubles psychiatriques associés à des troubles des systèmes transmetteurs du SNC.

11. Utilisation selon la revendication 10, pour laquelle les troubles psychiatriques sont choisis dans l'ensemble consistant en la dépression et la schizophrénie.

12. Utilisation selon l'une quelconque des revendications 1 à 11, qui comprend en outre un ou plusieurs agents ayant une activité neurotrophique ou des dérivés fonctionnellement actifs ou des parties de ces derniers.

13. Utilisation selon la revendication 12, pour laquelle l'agent est une cytokine.

14. Utilisation selon la revendication 13, pour laquelle la cytokine est choisie dans l'ensemble consistant en le GDF, le GDNF, le TGF, les activines, le BMP, le BDNF, le NGF, l'EGF, le CNTF et le FGF.

15. Utilisation d'un acide nucléique contenant une séquence nucléotidique codant pour la séquence primaire d'acides aminés d'une protéine comprenant au moins la séquence primaire d'acides aminés présentée sur la Figure 7B, la séquence primaire d'acides aminés présentée sur la Figure 8B ou les résidus d'acides aminés 14 à 111 de la séquence présentée sur la Figure 8B ou l'un de leurs fragments fonctionnellement actifs, ayant au moins un effet neurotrophique sur les neurones DAergiques ou leurs homologues fonctionnellement actifs ayant des substitutions conservatives d'acides aminés et ayant au moins un effet neurotrophique sur les neurones DAergiques et/ou d'un vecteur contenant au moins l'acide nucléique et/ou une protéine codée par l'acide nucléique et/ou d'un anticorps ou d'un fragment fonctionnel de ce dernier dirigé contre la protéine, pour préparer une trousse de diagnostic destinée à la détection des troubles neurodégénératifs chez les mammifères.

16. Utilisation selon la revendication 15, pour laquelle la protéine ou son dérivé fonctionnellement actif ou la partie de cette dernière, telle que définie dans la revendication 1, est un monomère, un dimère, un oligomère ou un hétérooligomère.

17. Utilisation selon la revendication 15 ou 16, pour laquelle la protéine telle que définie dans la revendication 1 assure une protection contre les événements neurodégénératifs.

18. Utilisation selon la revendication 17, pour laquelle l'événement neurodégénératif est provoqué par un dommage oxydatif et/ou un dommage occasionné par les radicaux libres et/ou les médiateurs et/ou les exécuteurs des programmes de mort neuronale.

19. Utilisation selon la revendication 18, pour laquelle les médiateurs des dommages provoqués par des radicaux libres sont choisis dans l'ensemble consistant en le fer, les donneurs de NO et d'autres donneurs de radicaux libres, et les médiateurs et exécuteurs des programmes de mort neuronale sont choisis dans l'ensemble consistant en les caspases et les membres pro- et anti-apoptotiques de la famille bcl-2.

20. Utilisation selon l'une quelconque des revendications 15 à 19, pour laquelle l'acide nucléique comprend au moins la séquence nucléotidique présentée sur la Figure 7A ou la séquence nucléotidique présentée sur la Figure 8A ou les nucléotides 40 à 333 de la séquence nucléotidique présentée sur la Figure 8A ou leurs mutants ayant une délétion, une insertion et/ou une substitution non nucléotide, lesdits mutants conduisant à l'expression d'un polypeptide fonctionnel ayant au moins un effet neurotrophique sur les neurones DAergiques.

21. Utilisation selon l'une quelconque des revendications 15 à 20, pour laquelle la protéine codée par l'acide nucléique comprend au moins la séquence primaire d'acides aminés présentée sur la Figure 7B ou la séquence primaire d'acides aminés présentée sur la Figure 8B ou les résidus d'acides aminés 14 à 111 de la séquence présentée sur la Figure 8B, ainsi que leurs homologues ayant des substitutions conservatives d'acides aminés.

22. Utilisation selon l'une quelconque des revendications 19 à 21, pour laquelle le mammifère est un humain.
